Europäisches Patentamt

European Patent Office

Office européen des brevets

(11) Publication number: **0 424 272 A2**

## EUROPEAN PATENT APPLICATION

(21) Application number: 90402948.5

(22) Date of filing: **19.10.90**

(51) Int. Cl.5: **A61B 17/22, A61B 17/36**

(30) Priority: **20.10.89 JP 273539/89**

(43) Date of publication of application:
**24.04.91 Bulletin 91/17**

(84) Designated Contracting States:
**AT CH DE ES FR GB IT LI NL SE**

(71) Applicant: **S.L.T. JAPAN CO, LTD.**
**402 Ebisutei Bldg., 8, Kagurazaka 5-chome**
**Shinjuku-ku, Tokyo 162(JP)**

(72) Inventor: **Daikuzono, Norio**
**381, Kofudai 4-chome**
**Ichihara-shi, Chiba-ken, 290-02(JP)**

(74) Representative: **Bloch, Gérard et al**
**2, square de l'Avenue du Bois**
**F-75116 Paris(FR)**

(54) Laser light irradiation apparatus.

(57) A laser light irradiation apparatus used for living tissues in a medical treatment. According to a preferred embodiment, the apparatus comprises a probe, plural number of optical fibers, whose tip ends are brought into contact with the back face of the probe and which are disposed to be parallel each other. Further, instead of a connecting metal device, which was used for placing the probe so as to be apart from the optical fibers in conventional apparatus, a laser light transmissible optical connecting member is used for connecting the transmissible substance and the optical fibers so that the tip end of the optical fiber is not contaminated and is not deteriorated, the laser light can be impinged into the probe from the optical fibers directly and its power level is not required to be adjusted at a laser light generator every treatment, and the fore end portion of this apparatus has a sufficient flexibility in its utility and its fabrication.

EP 0 424 272 A2

# LASER LIGHT IRRADIATION APPARATUS

## Background of the Invention

### 1. Field of the Invention

This invention relates to laser light irradiation apparatus, which irradiates laser light against living tissues of an animal such as a human body for use an incision, vaporization of the living tissues or a thermal therapy and in case of widening a narrow path of the living tissues such as a stricture part caused by cholesterol formed in the blood vessel of the human body.

### 2. Prior Art

Medical treatments such as incisions of living tissues of animal organisms by irradiation with laser light are conspicuous due to its ability of hemostasis in these days.

It had been the conventional method that the laser light was irradiated from the tip end of an optical fiber which is brought out of contact with the living tissues. But this method causes severe damage to the fore end portion of the optical fiber. Therefore, a method using a contact probe which has been utilized lately is as follows;

First, laser light is transmitted into an optical fiber, whose fore end portion locates adjacent to treated living tissues. Next, the laser light fed out from the optical fiber is impinged into an emitting probe, which is brought into or out of contact with the living tissues (hereafter "living tissue" is sometimes expressed by "tissue" only). Then, the laser light is emitted from the surface of the fore end portion of the probe to be irradiated against the living tissues. In this case, the probe should be brought into contact with the living tissues. The inventor developed many kinds of contact probes which are utilized for various purposes. By the utilization of the contact probes, following advantages can be obtained. One advantage is that the power level of the laser light irradiated against the tissues can be kept to be a desired level, as long as the power level of the laser light emitted from the probe can be adjusted uniformly. Another advantage is that laser light easily can be irradiated against a target area of a medical treatment as desired by an surgical operator. Therefore, the contact probes are used mainly in the medical treatment.

When laser light irradiation apparatus using this type of contact probe was utilized in the prior art, the tip end of an optical fiber was disposed so as to be apart from the back end face (an impinging face) of the probe. However, as so doing, the external surface of the fore end portion of the optical fiber was contaminated by splash of blood and the pieces of tissues and the like during an medical operation. Additionally, there is a space formed between the tip end of the optical fiber and the back end face of the probe, thus micro particles of dust in an operation room often infiltrate this space resulting in also contamination on the external surface of the fore end portion of the optical fiber.

This contamination influences the rate of laser light transmission from the optical fiber to the probe. Accordingly, treatments for the power level of the laser light supplied from a laser light generator are required; every operation or every preparation for the operation, the power level of the laser light generated in the laser light generator must be adjusted so that the suitable power level of the laser light can be emitted from the fore end portion of the probe, which requires much time. Furthermore, by this contamination, the fore end portion of the optical fiber is deteriorated. Additionally, since the optical fiber must be held so as to be apart from the probe, there must be a metal connecting device between the optical fiber and the probe. Accordingly, the fore end portion of this laser light irradiation apparatus lacks flexibility of utility and fabrication and the like.

## Summary of the Invention

It is therefore a main object of the present invention to provide laser light irradiation apparatus, in which the fore end portion of an optical fiber is not contaminated and whose fore end portion has a sufficient flexibility of its utility and its fabrication.

In order to solve the above mentioned problems, there is laser light irradiation apparatus comprising; a laser light transmissible substance or a probe; an optical fiber; and a laser light transmissible optical connecting member connecting the laser light transmissible substance and the optical fiber. In this case, as the laser light, Nd; YAG laser light is used preferably, however, other kinds of laser light such as argon-laser light can be used.

On the other hand, when the tip end of the optical fiber is brought into contact with the back face of the transmissible substance and the connecting member is provided so as to apply the external surface of the fore end portion of the optical fiber and the portion of the back face of the transmissible substance, which is other than the contacted portion by the tip end of the optical fiber, while the transmissible substance and the optical fiber are connected by the connecting member, the most of laser light emitted from the tip end of the optical fiber is not transmitted in the optical connecting member but impinged into the probe directly. Accordingly, this optical connecting member can be fabricated from a non-heat resistant material.

By provision of plural number of optical fibers at the back side of the probe, the laser light can be emitted uniformly, thus, it is possible to make the power level of laser light irradiated from the probe have a flat energy distribution as explained after.

By forming a through-hole along the axis of the probe and provision of plural number of optical fibers so as to be parallel each other at the back side of the probe, a flexible guide wire, by which the probe can be guided to be inserted, can be inserted through the through-hole. Therefore, when the probe should be inserted in a narrow passage of living body and a blood vessel and the like, this type of probe can be used efficiently. In this case, a wire for detecting the temperature of a target area can be inserted through the through-hole.

When the laser light is irradiated with low power level, a probe, which contains laser light scattering particles and which is fabricated from a transmissible synthetic resin material, can be used.

On the other hand, in case that the through-hole is formed as explained before, the laser light is emitted from the inner surface of the through-hole as well as the external surface of the probe. Therefore, a reflecting layer is preferably formed on the inner surface of the through-hole so that the laser light reaching the inner surface of the through-hole can be reflected there to be emitted from only the external surface of the fore end portion of the probe together with other laser light, which did not reach at the through-hole. This reflecting layer can be produced by gold-plating.

For the optical connecting member related to the present invention, a laser light transmissible synthetic resin material can be used. Then, the probe can be connected easily with the optical fiber by the above mentioned material, while this material is melted. Furthermore, this material is cheaper than the material of the conventional metal connecting device resulting in low cost. Additionally, it is possible to form the base portion and the probe of this laser light irradiation apparatus integrally from the synthetic resin material as a whole. Therefore, this apparatus can be produced with a flexibility of utility and fabrication.

On the other hand, in order to irradiate the laser light from the whole external surface of the probe uniformly against the tissues of the target area, a surface layer is preferably formed on the surface of the probe. This surface layer contains laser light absorbing particles, light scattering particles, which have a larger refractive index than that of the material of the probe or that of the above mentioned synthetic resin material and a laser light transmissible material as a binder. Therefore, the laser light can be scattered on the surface layer to give a uniform laser light irradiation.

Alternatively, for the uniform laser light irradiation, another surface layer can be formed on the surface of the probe. In this modified surface layer, a number of light scattering particles are not melted completely, thus, the layer can not be homogenous; they are partially melted and partially remain to have particle-shapes to form a stratified surface layer.

As so doing, the laser light is irradiated against the tissues after being impinged into and being emitted from the light scattering particles with refractions in the surface layer. However, if the light scattering particles were melted completely to form a homogenous layer, the laser light could not be impinged into and emitted from the particles with any refraction, thus, there would be no scattering effect, which can be produced by the above mentioned stratified surface layer.

According to the present invention, since the probe and the optical fiber are connected each other by means of the optical connecting member, the fore end portion of the optical fiber is not contaminated, and the power level of the laser light emitted from the laser light generator is not required to be adjusted in accordance with the suitable energy of the laser light to be irradiated against the tissues resulting in efficient operation. Further, the fore end portion of the optical fiber is not deteriorated, thus, this apparatus can be used for longer time than conventional apparatus. Additionally, metal connecting device, which was used in conventional apparatus for placing the probe being apart from the tip end of the optical fiber, is not required in apparatus of the present invention. Then, in stead of the metal connecting device, for example, the optical connecting member fabricated from the synthetic resin material is used. As a result, the fore end portion of this apparatus can be produced with the sufficient flexibility in its utility and its fabrication and the like.

Further objects and advantages of the present invention will be apparent from the following description, reference being had to the accompanying drawing wherein preferred embodiments of the present invention are clearly shown.

**Brief Description of the Drawings**

Fig. 1 is a longitudinal sectional view of irradiation apparatus in first embodiment related to the present invention;

Fig. 2 is a sectional view taken on line II-II of Fig. 1;

Fig. 3 is a longitudinal sectional view of an important part of irradiation apparatus, which is modified from the first embodiment;

Fig. 4 is an illustration showing operation of irradiation apparatus being inserted into a blood vessel;

Fig. 5 is a longitudinal sectional view of an important part of irradiation apparatus carrying out a local thermal therapy for cancer tissues;

Fig. 6 is a schematic illustration for a temperature distribution with the apparatus of Fig. 5;

Fig. 7 is a plan view of the temperature distribution of Fig. 6;

Fig. 8 is a temperature distribution with conventional apparatus;

Fig. 9 is a longitudinal sectional view of irradiation apparatus in another embodiment;

Fig. 10 is a longitudinal sectional view of irradiation apparatus in still another embodiment

Fig. 11 is a side view of Fig. 10 from a left-side on this figure;

Fig. 12 is a longitudinal sectional view of further another embodiment;

Fig. 13 is a side view of Fig. 12 from a left-side gn this figure;

Fig. 14 is a longitudinal sectional view of an embodiment of irradiation apparatus in a surgical treatment.

## Description of the Preferred Embodiment

Now, the present invention is described more particularly with several kinds of embodiments.

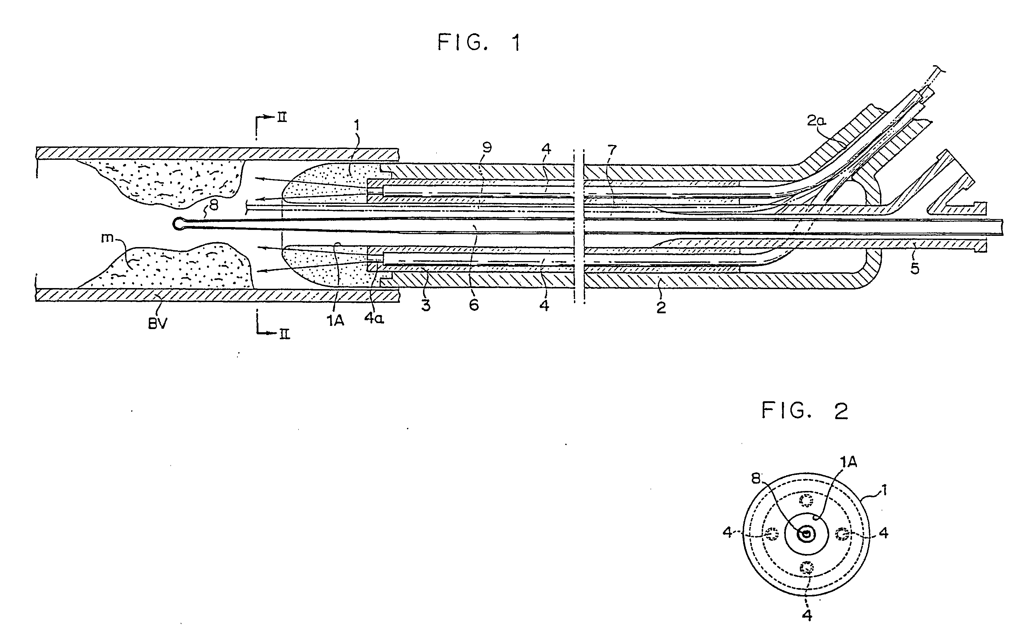

Figs. 1 and 2 show a first embodiment mainly for an angio-plasty. A probe 1 is fabricated from a synthetic resin material or ceramics and so on. The external surface of fore end portion of the probe 1 is rounded off at its circumference in order to decrease the friction caused by proceeding of the probe in a blood vessel at the inner wall of the vessel. A main tube 2 is provided so as to be coaxial with the probe 1 and is fabricated from a flexible material such as the resin of tetrafluorethylene and the like, and is engaged and connected to the probe 1.

In the main tube 2, plural number of, for example four in Fig. 1, optical fibers 4 are provided so as to be parallel each other and surround the axis of the probe 1 or the axis of the main tube 2. These optical fibers are connected optically with a laser light generator (not shown). Each fore end portion of the optical fiber 1 is exposed to be a core 4a, although it in not always required to be exposed to be a core. Each core 4a is brought into contact with the back face or the impinging face 1a of the probe 1. Each optical fiber 4 is inserted into the main tube 2 from an inserting hole 2a. Then, each optical fiber 4 is supported by the probe 1 and the main tube 2 through an optical connecting member 3. For this connecting, the connecting member 3 melted previously is painted on the external surface of the fore end portion of the optical fiber 4 to the back face of the probe 1, while the core 4a of each optical fiber 4 is brought into contact with the back face of the probe 1.

On the other hand, a through-hole 1 A is formed to go through along the axis of the probe 1 to communicate with the inner through-hole of the connecting member 3. A conductive tube 5 is provided in the main tube 2 so as to be projected from the back end of the main tube 2. The fore end portion of the conductive tube 5 is inserted into the inner side of the connecting member 3. A flexible guide wire 6 is inserted through the inner side of the connecting member 3 and continuously the through-hole 1A so as to be projected forward from the through-hole 1A of the probe 1. The base portion of the guide wire 6 is coated by a synthetic resin coating such as the resin of tetrafluorethylene. The fore end portion of the guide wire 6 is tapered gently and is totally covered with a gold plated layer 8. A lead wire 9 for detecting a temperature, which is used not for the angio-plasty but for a thermal therapy, can be inserted through this apparatus.

In this type of laser light irradiation apparatus, the laser light fed from the laser light generator goes through the optical fiber 4 to reach its tip end. Continuously, the laser light emitted from its tip end is impinged into the probe 1 directly. Then, the laser light goes through inner side of the probe 1 to be emitted and irradiated against the treated tissues. On the other hand, for a medical operation, this laser light irradiation apparatus is used as follows;

First, out of the human body, the guide wire 6 is inserted through the apparatus. Next, the guide wire 6 is further inserted into the treated blood vessel BV so that the tip end of the guide wire 6 is proceed further than a stricture part m, which will be burnt off by laser light irradiation.

Then, this apparatus other than the guide wire 6 is inserted in the blood vessel BV along the guide wire 6 so as to proceed until the tip end of the probe 1 is adjacent to the stricture part m. The laser light is fed into each optical fiber 4 to be emitted from its tip end. The emitted laser light from the optical fiber 4 impinges into the back face of the probe 1 and go through therein. Then, the laser light is emitted from the probe 1 mainly from the peripheral external surface of its tip end. Finally, the laser light is irradiated against the stricture part m.

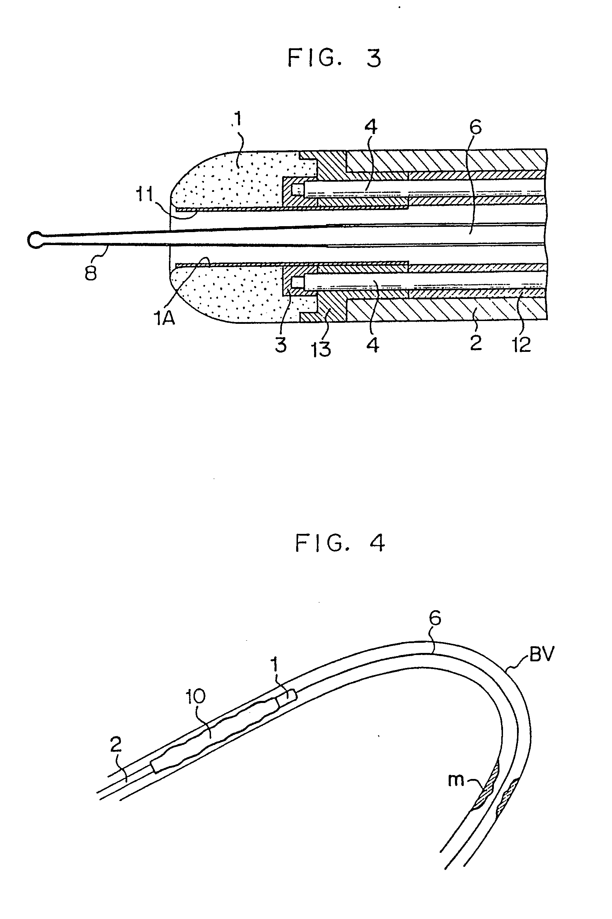

By the laser light irradiation, the stricture part m is burnt off to wide the inside of a blood vessel BV. In this case, if desired, as shown in Fig. 4, pressurized air or pressurized liquid is sent into a balloon 10 connected between a probe 1 and a main tube 2, thus, the balloon 10 is expanded and press the stricture part m. As so doing, together with the above mentioned burning off the inside of the blood vessel BV by the laser light irradiation, the stricture part m can be broken mechanically.

As shown in Fig. 1, in the present invention, the laser light is emitted from the peripheral external surface of the tip end of the probe 1. Therefore, the laser light is irradiated efficiently against the stricture part m formed on the inner wall of the blood vessel BV. Accordingly, the stricture part m can be burnt off sufficiently even if the power level of the laser light is low.

When the laser light is irradiated against the stricture part m, the laser light is irradiated against also the projecting part of the guide wire 6. However, since the surface of the fore end portion of the guide wire 6 is coated by the gold plated layer 8 as explained before, this surface can be prevented from being damaged.

As shown in Fig. 3, if desired, a reflecting sleeve-like tube 11 fabricated from a thin metal is provided on the inner side surface of a through-hole 1A and a laser light reflecting layer such as a gold plated layer is formed on the surface of the sleeve-like tube 11. Accordingly, laser light is reflected on the gold plated layer and the laser light irradiated toward the axis of a probe 1 can be decreased.

On the other hand, in the embodiment of Fig. 3, the base portion of the optical fiber 4 is held by a holding member 12 fabricated from a suitable synthetic resin material, while the fore end portion of the optical fiber 4 is held by a metal holder 13. Then, only the fore end portion of each optical fiber 4 is supported by a connecting member 3. Additionally, as shown in this figure, the fore end portion of each optical fiber 4 can be disposed in the optical connecting member 3 so as to be apart from the back face of the probe 1. In this case, the laser light emitted from the tip end of the optical fiber 4 is impinged into the connecting member 3 and goes through its inner side before being impinged into the probe 1.

For the optical connecting member related to the present invention, any material, which is laser light transmissible, can be used. However, due to its connecting or adhering ability and its flexibility, a synthetic resin material is generally preferable to be used. For example, there are acrylic synthetic resin, polycarbonate resin, polyester resin, epoxy resin, and conventional ultraviolet rays hardening resin and the like. Alternatively, a ceramic material such as low melting point glass can be used.

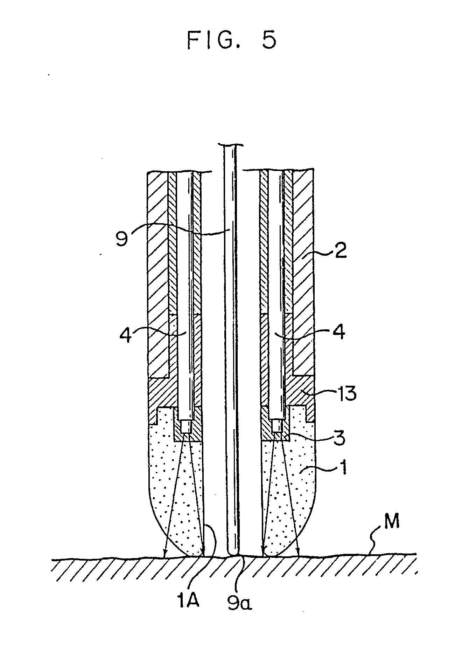

These above mentioned apparatus of Figs. 1 and 3 are used for also a thermal therapy efficiently. As shown in Fig. 5, a lead wire 9 for detecting a temperature having a thermocouple 9a at its tip end is pushed into the cancer tissues M, while a probe 1 is brought into contact with the surface of the cancer tissues M. Then, laser light having a low power level is irradiated against the tissues M from each optical fiber 4 through the probe 1. In this case, the power level of the irradiated laser light should be controlled so as to keep the tissues M at the temperature of about 42-44°C.

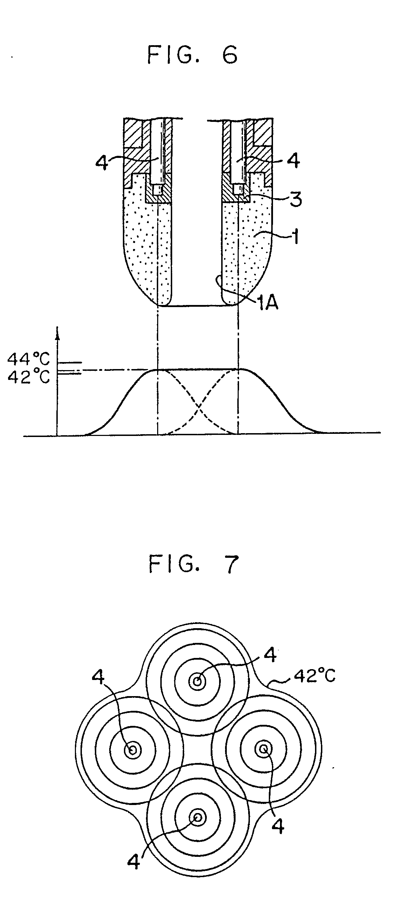

In this way, by laser light irradiation from the peripheral external surface of the tip end of the probe 1 against the cancer tissues M, as shown in the temperature distribution diagrams of Figs. 6 and 7, the temperature can be controlled so as to be uniform in a broad area, comparing with a temperature distribution diagram produced by laser light irradiation with conventional laser light irradiation apparatus in which single optical fiber is disposed so as to be coaxial with and so as to be at the back side of the single probe P.

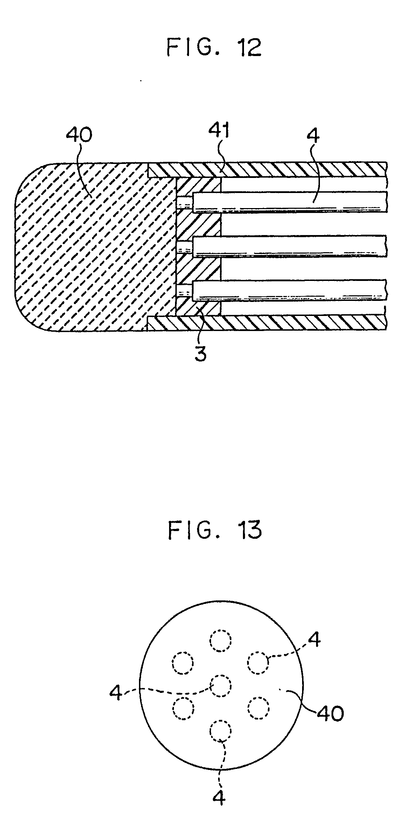

In the embodiment of Fig. 5, the through-hole 1A is not always necessary like another embodiment in Fig. 12. However, by the provision of the through-hole 1A and the plural number of optical fibers 4 at the back side of the probe 1, the laser light, which reaches at the inner side surface of the through-hole 1A, is partly refracted to penetrate through the through-hole 1A, however, mainly reflected to go toward the tip end of this apparatus. Comparing an apparatus having no through-hole, in case of the apparatus having the through-hole, the rate of the emitted laser light from the peripheral external surface of the tip epd of the probe 1 is more increased, thereby, more uniform temperature distribution is obtained.

According to the present invention, further various types of probes can be provided.

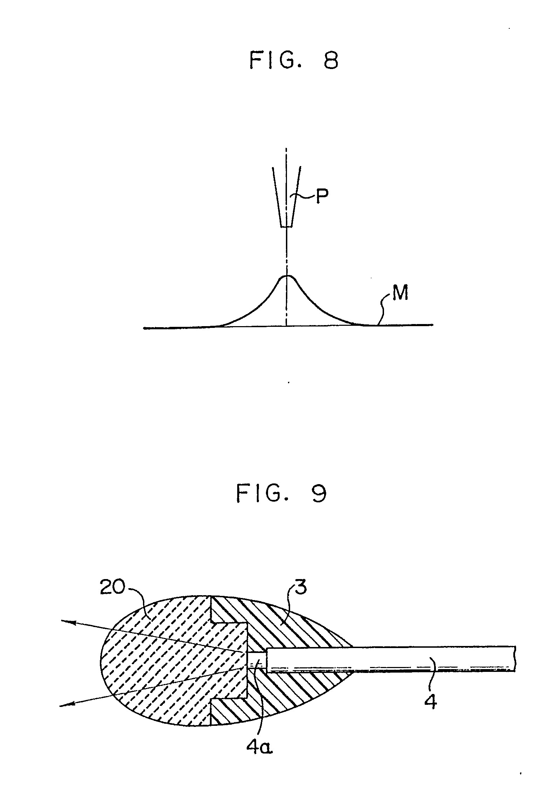

For example, Fig. 9 shows a most simplified structure. In this embodiment, an optical fiber 4 is connected to a probe 20 having a suitable shape by a connecting member 3. This apparatus can be applied to be connected to an endoscope.

When the power level of the laser light emitted from the probe is low, the power level of the laser light impinged into the connecting member 3 is extremely low. On the other hand, when the laser light is emitted with a high power level, the laser light is impinged into the connecting member 3 with a high power level resulting in being heated and damaged.

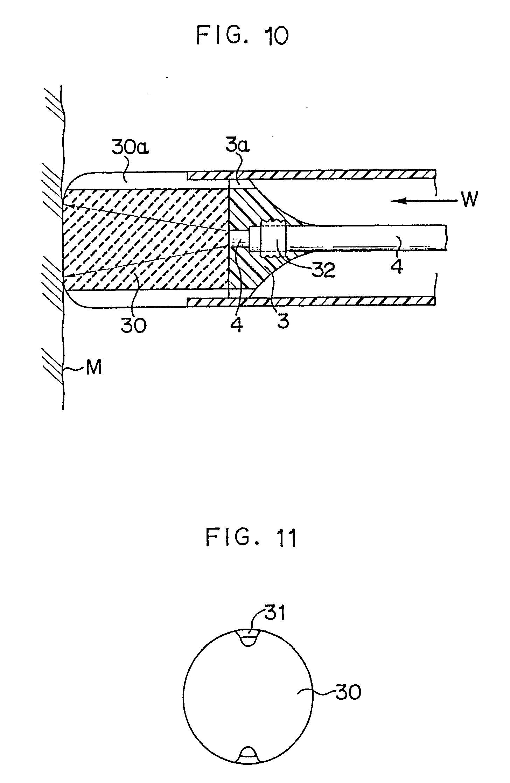

Therefore, for the laser light emitted with high power level, apparatus of Figs. 10 and 11 can be preferably used.

In this embodiment, an optical fiber 4 is connected to a probe 30 by a connecting member 3 and a flexible tube 31 is connected to the back end of the probe 30 so as to include the optical fiber 4. Pure air or a physiological salt solution W is charged in the tube 31. Each pair of notch-grooves 3a, 30a are formed on the connecting member 3 and the probe 30 so as to be communicated with each other. Then, the pure air or the physiological salt solution W is flown out from the notch-grooves 3a, 30a, while it cools the connecting member 3 in order to prevent the connecting member 3 being heated and damaged.

On the other hand, in this embodiment, there may be a fear that the optical fiber 4 is removed from the probe 30 due to the destructure of the connecting member 3 caused from an unexpected shock and the like. Accordingly, for example, a sleeve-like small cover 32, which is fabricated from metal or a synthetic resin material, can be provided so as to surround the fore end portion of the optical fiber 4 as shown in this figure. This cover 32 is adhered or caulked to be fixed on the optical fiber 4 so as not to be removed, and the external surface of the cover 32 is formed to be rough before connecting the probe 30 and the optical fiber 4 by the connecting member 3. Finally, the optical fiber 4 is prevented from being removed from the probe 30.

Figs 12 and 13 show an embodiment of irradiation apparatus, in which plural number of, for example seven, optical fibers 4 are disposed so as to be parallel each other and to be connected to a probe 40 having no through-hole by a connecting member 3. Numerical number 41 indicates a tube. Also in this embodiment, laser light is irradiated in the substantially same manner as that of the above mentioned embodiments.

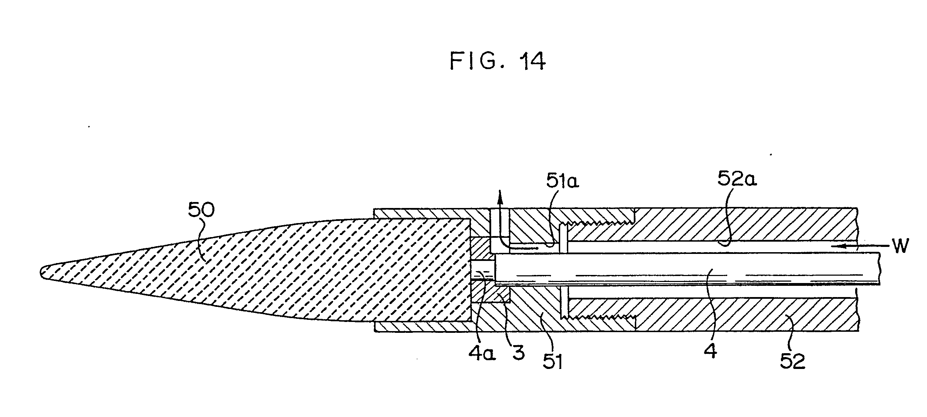

These probes having the structures described in the above embodiments are utilized in the field of an internal treatment. Then, Fig. 14 shows an embodiment of a probe utilized in the field of a surgical treatment. In this embodiment, a probe 50 is supported by a metal coupling 51. Then, the fore end portion of an optical fiber 4 is supported by the coupling 51 and a holder 52, which is connected removably to the coupling 51. Additionally, the tip end of the optical fiber 4 and the probe 50 are connected optically by a connecting member 3.

Further, a physiological salt solution W is charged through inner side. of the holder 52. Continuously, the physiological salt solution W is flown out from a through-hole 52a in the holder 52 and a throuh-hole 51b in the coupling 51, while it cools the connecting member 3.

For the probes in these embodiments, a ceramic material such as quartz, sapphire and cilica and the like are generally used. However, if laser light is irradiated with a low power level and the laser light is emitted from a whole of external surface of the probe, the probe, which contains laser light scattering particles and which is fabricated from the laser light penetrating synthetic resin material, also can be used.

As this synthetic resin material, the materials used for the above mentioned connecting member can be used. For example, there are silicone resin, acrylic resin (more preferably, methyl metha-acrylate resin), carbonate resin, polyamide resin, polyethylene resin, urethane resin, polyester resin and the like, more preferably, thermoplastic synthetic resin. For the laser light scattering particles, the material, which has a larger refractive index for the laser light than that of the above mentioned synthetic resin material of the probe, is used, for example, a natural or an artificial material such as diamond, sapphire, quartz material, single crystal zirconium oxide, laser light transmissible synthetic resin having heat resistance (it is needless to say that it is different from the above mentioned synthetic resin material of the probe), laser light reflective metal (such as gold, aluminum and the like), and the particles on whose surface the above mentioned laser light reflective metal are coated to be a compound material.

On the other hand, if desired, the probe can contain laser light absorbing particles such as carbon, graphite, iron oxide, manganese dioxide and the like together with the scattering particles. As so doing, while the laser light is scattered in the probe to be emitted from the probe, the laser light is impinged on the absorbing particles to generate heat energy to give a large heating effect.

The above mentioned probes fabricated from synthetic resin material can be fabricated by moulding to be a desired shape from the synthetic resin material, which is in a melted state and into which the scattering particles are dispersed.

In the resulted laser light irradiation apparatus, the laser light impinged into the probe is emitted from the external surface of the probe, while the laser light repeats to be refracted on impinging on the scattering particles to give a substantially uniform laser light irradiation against the tissues from the external surface of the probe.

Further, if desired, a surface layer might be formed on the surface of each probe having a structure above desdribed to give a large scattering effect.

This surface layer contains light scattering particles, which have the larger refractive index than that of the material of the probe or that of the above mentioned ceramic or synthetic resin material. For example, sapphire, silicon dioxide, aluminum oxide and the like are used as the scattering particles. Then, the surface layer contains also laser light absorbing particles, which can be included in the probe as described before, such as carbon and the like. Finally, the surface layer contains a binder, which sticks the particles to each surface and forms a film on the surface.

Due to the surface layer, the laser light is scattered by the light scattering particles, further, when the laser light impinges on the laser light absorbing particles, the greater part of the energy of the laser light is converted to heat energy.

As so doing, as the vaporization of the tissues is accelerated, the tissues can be incised with the laser light having the low power level of incident laser energy into the probe. Therefore, when the tissues are incised, the probe can be moved rapidly. Further, since the power level of the incident laser energy into the probe is required to be low, the medical operation can be carried out in short time with a cheap and small scaled laser light generator.

On the other hand, referring to the surface layer, if a dispersion containing the laser light absorbing particles and the light scattering particles was coated on the surface of the probe, after a vaporization of a dispersion medium, the contact of this probe with the tissues or other substances would cause a damage to the surface layer, because the both kinds of particles are attached to the surface of the probe only by physical adsorptive power.

Therefore, by the binder which sticks the laser light absorbing particles and the light scattering particles to the surface of the probe, an adhesion of the surface layer to the probe is enhanced. In this case, the binder is preferably made of light transmissible particles such as synthetic resin particles or ceramic particles such as quartz particles and the like. For forming the film, when the synthetic resin particles are used as the material of the binder, the particles should be melted, or when the ceramic particles having a higher melting point than that of the probe are used, the surface of the probe should be melted.

Alternatively, another surface layer can be formed. In this modified surface layer, a number of light scattering particles are not melted completely, thus, the layer can not be homogenous; they are partially melted and partially remain to have particle-shapes to form a stratified surface layer. For forming this surface layer, the laser light absorbing particles and the light scattering particles are dispersed in a volatile liquid such as alcohol. Then, the probe is dipped into the dispersion. After pulling the probe from the dispersion, the probe is heated to a temperature, which is adjacent to the melting point of the light scattering particles. Continuously, the scattering particles are partially melted to be attached to the surface of the probe. As a result, the stratified surface layer can be formed.

Further, by forming a rough surface on the surface of the probe or by forming the above mentioned surface layer on the rough surface, since the laser light is scattered on the rough surface, the laser light can be irradiated more effectively.

While preferred embodiments have been described, it is apparent that the present invention is not limited to the specific embodiments thereof.

## Claims

1. Laser light irradiation apparatus comprising; a laser light transmissible substance;
an optical fiber; and
a laser light transmissible optical connecting member, which connects said laser light transmissible substance and said optical fiber.

2. Apparatus according to claim 1, wherein the tip end of said optical fiber is brought into contact with the back face of said transmissible substance and said connecting member is provided so as to apply the external surface of the fore end portion of said optical fiber and the portion of said back face of said transmissible substance, which is other than the contacted portion by said tip end of said optical fiber, while said transmissible substance and said optical fiber are connected by said connecting member.

3. Laser light irradiation apparatus comprising;
a laser light transmissible substance;
plural number of optical fibers disposed so as to be parallel each other; and
an optical connecting member connecting said laser light transmissible substance and said optical fibers.

4. Apparatus according to claim 3, wherein a through-hole is formed along the axis of said laser light transmissible substance and said plural number of optical fibers are disposed so as to be parallel each other and so as to be at the back side of said transmissible substance.

5. Apparatus according to claim 4, wherein a flexible guide wire for guiding said transmissible substance is inserted through said through-hole.

6. Apparatus according to claim 5, wherein a lead wire for detecting a temperature is inserted through said through-hole.

7. Apparatus according to claim 1, wherein said transmissible substance contains laser light scattering particles and is fabricated from a laser light transmissible synthetic resin material.

8. Apparatus according to claim 1, wherein a through-hole is formed along the axis of said laser light transmissible substance and a reflecting layer is formed on the inner surface of said through-hole.

9. Apparatus according to claim 8, wherein said reflecting layer is a gold plated layer.

10. Apparatus according to claim 1, wherein said optical connecting member is fabricated from a laser light transmissible synthetic resin material.

11. Apparatus according to claim 1, wherein a surface layer is formed on the surface of said laser light transmissible substance, said surface layer contains laser light absorbing particles, light scattering particles which have a larger refractive index than that of said transmissible substance and a laser light transmissible material as a binder.

12. Apparatus according to claim 1, wherein a surface layer is formed on the surface of said laser light transmissible substance, said surface layer contains a number of light scattering particles being partly melted and partly remain to have substantially particle-shapes so as to form a stratified layer.

FIG. 1

FIG. 2

EP 0 424 272 A2

FIG. 3

FIG. 4

FIG. 5

FIG. 6

FIG. 7

FIG. 8

FIG. 9

FIG. 10

FIG. 11

FIG. 12

FIG. 13

FIG. 14